# EUROPÄISCHE PATENTSCHRIFT

(11) **EP 1 470 792 B1**
(45) Veröffentlichungstag und Bekanntmachung des Hinweises auf die Patenterteilung: **14.09.2005**
(21) Anmeldenummer: 03009327.2
(22) Anmeldetag: 24.04.2003
(51) Int. Cl.: A61B 19/00, A61B 5/103

(54) **Abstandsmessgerät für Pedikelschrauben**
Distance measurement instrument for pedical screws
Instrument de mesure de la distance entre des vis pédiculaires

(43) Veröffentlichungstag der Anmeldung: 27.10.2004
(73) Patentinhaber: Zimmer GmbH, 8404 Winterthur (CH)
(72) Erfinder: Casutt, Simon, 9200 Gossau (CH); Meier, Nimrod, 8200 Schaffhausen (CH); Huber, Marc, 8488 Turbenthal (CH)
(74) Vertreter: Manitz, Finsterwald & Partner GbR

(56) Entgegenhaltungen:
- US-A- 3 815 247
- US-A- 5 188 121
- US-A- 5 329 933

## Beschreibung

Die Erfindung handelt von einem Abstandsmessgerät für Pedikelschrauben mit zwei sich an einer Drehachse kreuzenden Schenkeln, die mit ihren Enden an zwei in benachbarten Rückenwirbeln eingedrehte Pedikelschrauben ansetzbar sind, wobei ein erster Schenkel an der entgegengesetzten Seite zu seinem Ende in einen Querbalken übergeht, welcher mit einem bogenförmigen Skalenteil versehen ist, wobei der zweite Schenkel einen über den Querbalken vorstehenden Vorsprung zur Verstellung aufweist und als Zeiger ausgebildet ist, um den Abstand zwischen den Pedikelschrauben mit dem Zeiger an einer Skala des Skalenteils anzuzeigen. Eine derartige Abstandsmessung dient der Festlegung der Länge von Bauelementen, welche die Distanz zwischen den beiden Pedikelschrauben überbrücken sollen.

Die Firma Centerpulse Orhopedics Ltd. (Altgasse 44, CH-6340 Baar) führt in ihren Instrumenten ein Abstandsmessgerät wie es in Figur 2 als "Prior Art" dargestellt ist. Ein derartiges Messgerät gestattet es, den Abstand zwischen zwei eingedrehten Pedikelschrauben zu messen, wenn die Schraubenköpfe gut sichtbar sind und so das Anliegen der Enden sichtbar kontrolliert werden kann. Bei nicht direkt sichtbaren Köpfen der Pedikelschrauben, ist eine Abstandsmessung recht schwierig.

Ein weiteres Abstandsmessgerät ist aus der Patentschift US-A-5,329,933 bekannt.

Aufgabe der vorliegenden Erfindung ist es, diesen Zustand im Hinblick auf Operationstechniken zu verbessern, die nur ein kleines, minimalinvasives Operationsfeld vorsehen. Diese Aufgabe wird mit den Kennzeichen des unabhängigen Anspruchs 1 dadurch gelöst, dass der zweite Schenkel in einen Zeiger und eine parallel dazu verlaufende Biegefeder unterteilt ist, an welcher ein ausgeprägter Daumengriff befestigt ist, und dass der Querbalken als Handgriff geformt ist, um über die Biegefeder eine Spreizkraft an den Enden zu erzeugen, welche über eine mit dem Daumengriff verbundene Skala relativ zum Zeiger ablesbar ist.

Diese Anordnung hat den Vorteil, dass während dem Ablesen des Abstandes der Enden unter Vorspannung eine taktile Rückmeldung am Daumengriff für das Anliegen der Enden vorliegt. Im weiteren hat sich gezeigt, dass durch eine vorgegebene Vorspannkraft beim Spreizen eine anfängliche Nachgiebigkeit zwischen zwei Wirbeln mit einbezogen werden kann, um die Einbaulänge eines Abstützelementes zu bestimmen.

Mit den Merkmalen der abhängigen Ansprüche 2 bis 10 sind vorteilhafte Weiterbildungen der Erfindung aufgezeigt.

Eine robuste und wenig anffällige Konstruktion für das Abstandsmessgerät ergibt sich, wenn der zweite Schenkel mit Zeiger und Biegefeder beidseitig in einem Längsschlitz des Querbalkens geführt ist. Im bogenförmigen Skalenteil ist eine ebenfalls bogenförmige Nute für einen Nutenstein eingearbeitet, welcher als Schleppzeiger ausgebildet ist und an einem vorstehenden Nocken durch den Zeiger am zweiten Schenkel mitgenommen wird. Der eigentlich mit dem Nutenstein verbundene Zeiger bleibt an der Skala des bogenförmigen Skalenteils als gespeicherter Wert stehen unabhängig davon, ob der Zeiger vom zweiten Schenkel, beispielsweise zum Ausfahren von Zentrierelementen aus zentrierenden Bohrungen an den Pedikelschrauben, seinen Abstand verringern muss.

Die taktile Rückführung bezüglich der erreichten Abstandsposition zwischen zwei Pedikelschrauben kann wesentlich verbessert werden, wenn an den Enden der Schenkel passende Zentrierungen angebracht sind, die sich in der Spreizrichtung an dem Kopf einer Pedikelschraube oder an mit dieser verbundenen Elementen zentrieren. So kann das Ende eines Schenkels in Spreizrichtung mit einer Nase in Form einer vorspringenden Halbkugel oder eines Konusstumpfes ausgebildet sein, um sich in einer Bohrung des Kopfes zu zentrieren. Solange ein solcher Schenkel in Spreizrichtung unter Vorspannung steht, können die zentrierenden Elemente nicht abrutschen. Gleichzeitig entsteht mit leichtem Rütteln am Schenkel, die taktile Rückmeldung, dass das Ende zentriert an seinem vorgesehenen Platz ist. Auch bei Pedikelschrauben, bei denen schon ein Band im Kopf eingezogen ist, lässt sich eine Zentrierung am Austritt des Bandes mit einem Schenkelende durchführen, welches gabelförmig ist.

Im weiteren ist es vorteilhaft die Enden der beiden Schenkel in Form von seitlich versetzten Vorsprüngen so abzukröpfen, dass die Schenkel selbst und der Rest des Abstandsmessgerätes seitlich versetzt zu der durch die beiden Pedikelschrauben aufgespannten Ebene liegen. Dies ermöglicht es Werkzeuge und Zentrierelemente wie sie in einer Parallelanmeldung beschrieben sind, unabhängig weil räumlich versetzt angeordnet, zu benutzen. Wenn Instrumente oder Zentrierteile benutzt werden, die senkrecht von oben am Kopf einer kaum noch sichtbaren Pedikelschraube angreifen, dann können die Enden der Schenkel zusätzlich in Spreizrichtung eine Führungsgabel aufweisen, mit der die Enden auf ihrem Weg zum Kopf geführt sind bis ein eigentliches Zentrieren am Kopf möglich ist.

Weitere Vorteile in der Handhabung entstehen, wenn die Skala für die Ablesung der Vorspannung an einem Bügel angebracht ist, welcher den Zeiger mit Spiel hintergreift, um Überbeanspruchung der Biegefeder zu verhindern und um die Vorspannung innerhalb dieses Spiels abzulesen. Wenn man die Schenkel des Messgerätes aus Metall (z.B. aus einer nicht rostenden Stahllegierung) und den Querbalken, welcher den zweiten Schenkel führt, aus Kunststoff ausführt, ergibt sich ein geringes Eigengewicht und eine günstige und lautlose Materialpaarung für die Führung. Ausserdem ist es möglich einen ganzen Satz von unterschiedlichen auf die Schenkel aufsteckbaren Enden als Adapterstücke vorzusehen, welche eine Gabelfom in Richtung der Schenkelachse, eine zentrierende Kugel- oder Konusform in Spreizrichtung, einen seitlichen Versatz und / oder Gabelform in Spreizrichtung aufweisen.

Im folgenden wird die Erfindung anhand von Ausführungsbeispielen beschrieben. Es zeigen:
- Fig.1:: Schematisch einen Schnitt mit zwei in benachbarten Wirbeln eingedrehten Pedikelschrauben;
- Fig.2:: Schematisch eine Ansicht eines bekannten Abstandsmessgerätes für die Pedikelschrauben aus Figur 1;
- Fig.3:: Schematisch eine Seitenansicht eines erfindungsgemässen Abstandsmessgerätes;
- Fig.4:: Schematisch eine Seitenansicht eines weiteren Abstandsmessgerätes analog zu Figur 3;
- Fig.5:: Schematisch eine Ansicht schräg von oben auf das Abstandsmessgerät von Figur 4;
- Fig.6:: Schematisch einen Ausschnitt am Ende eines zweiten Schenkels, der mit einem versetzten Ende an einer Pedikelschraube mit eingedrehten Zentrierteil anliegt;
- Fig.7:: Schematisch ein Ende des zweiten Schenkels von Figur 6 mit einem Halbkugelvorsprung für die Zentrierung an einer Durchgangsbohrung der Pedikelschraube;
- Fig.8:: Schematisch einen Ausschnitt am Ende eines ersten Schenkels, der mit seinem versetzten Ende, welches sich als Gabel an einem durch die Pedikelschraube durchgezogenen Band zentriert, am Kopf der Pedikelschraube anliegt; und
- Fig.9:: Schematisch einen stark vergrösserten Ausschnitt auf den Skalenteil und die Anzeigen des Abstandsmessgerätes von Figur 4.

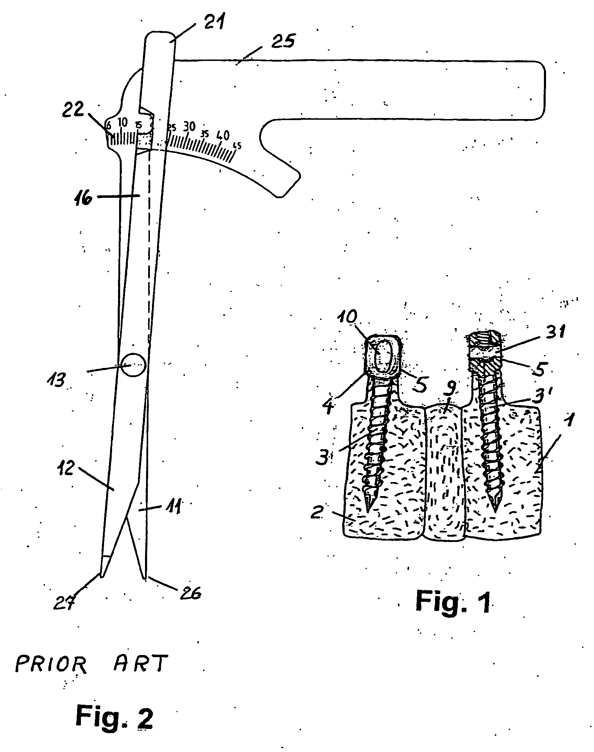

Die Figuren 1 und 2 zeigen, wie bisher eine Abstandsmessung zwischen zwei Pedikelschrauben vorgenommen wurde. Zwei Pedikelschrauben 3, 3' sind in zwei benachbarte Rückenwirbel 1, 2 eingeschraubt, welche durch eine Bandscheibe 9 getrennt sind. Jede der Pedikelschrauben 3, 3' besitzt einen Kopf 4, mit Stirnflächen 5, an denen später Überbrückungsteile angelegt werden. Die Köpfe 4 sind so ausgerichtet, dass ihre Durchgangsbohrungen 31 fluchten. An den Köpfen 4 sind seitlich Kerben 10 an denen weitere Instrumente ausgerichtet werden können. Ein bisher bekanntes Abstandsmessgerät besteht aus zwei sich an einer Drehachse 13 kreuzenden Schenkeln 11, 12 deren Enden 26, 27 aufspreizbar sind, um beispielsweise den Abstand zwischen den beiden Pedikelschrauben 3, 3' abzugreifen und mit dem zweiten Schenkel 12 als Zeiger 16 an einer bogenförmigen Skala 22 abzulesen, welche an einem Querbalken 25 des ersten Schenkels 11 angebracht ist. Dazu wird der Zeiger 16 mit einem Vorsprung 21 bewegt.

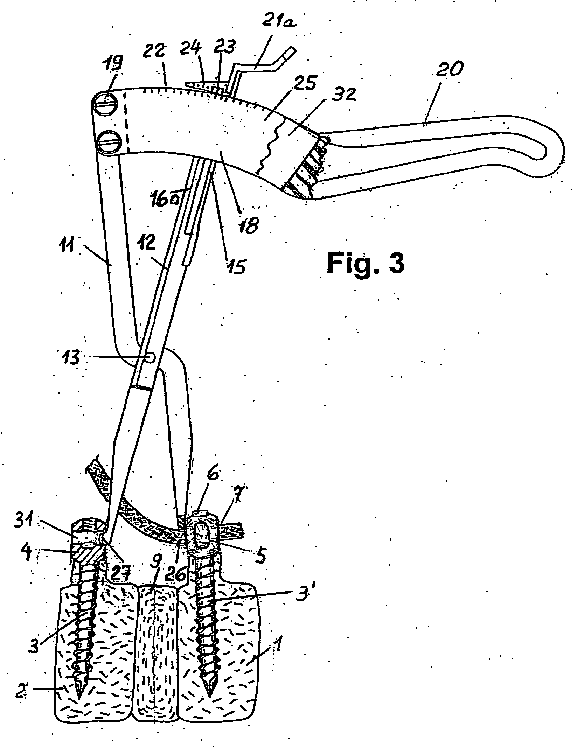

Ein erstes Beispiel der Erfindung ist in Figur 3 gezeigt. An zwei benachbarten Wirbeln 1, 2 mit Pedikelschrauben 3, 3' liegt ein Abstandsmessgerät mit seinen Schenkeln 11, 12 an. Der erste Schenkel 11 besitzt ein gabelförmiges Ende 26, mit dem er an der Pedikelschraube 3' anliegt und mit dem er gleichzeitig an einem aus dem Kopf der Pedikelschraube herausragenden Band 7 zentriert ist. Das Band 7 ist mit einer Klemmschraube 6 in einer Durchgangsbohrung 31 fixiert. Das gabelförmige Ende 26 liegt unter leichter Vorspannung am Kopf der Pedikelschraube 3' an. Die zweite Pedikelschraube 3 hat ebenfalls eine Durchgangsbohrung 31 in ihrem Kopf 4 in welche das Band 7 zu einem späteren Zeitpunkt eingezogen wird. An dieser Durchgangsbohrung 31 zentriert sich der zweite Schenkel 12 mit seinem Ende 27, welches einen in Spreizrichtung vorstehenden Vorsprung aufweist.

Die beiden Schenkel 11, 12 kreuzen sich an einer Drehachse 13. Am ersten Schenkel 11 ist entgegengesetzt zu seinem Ende 26 ein Querbalken 25 aus Kunststoff angeschraubt, welcher zunächst als kreisbogenförmig gekrümmter Skalenteil 18 mit der Drehachse 13 als Mittelpunkt seitlich wegsteht und in einen Griffteil 20 übergeht. Die beiden Schenkel 11, 12 sind aus Metall beispielsweise aus einem rostfreien Stahl hergestellt. Der erste Schenkel ist mit Schrauben 19 am Querbalken 25 befestigt. Der zweite Schenkel 12 ist in einem Längsschlitz 32 des Querbalkens 25 geführt und in seiner Längsrichtung in zwei Komponenten aufgeteilt, einen Zeiger 16a und eine Biegefeder 15, die beide im Längsschlitz 32 gefangen sind. Die Biegefeder 15 verläuft parallel zum Zeiger 16a, ist beispielsweise mit Schrauben (nicht gezeigt) am zweiten Schenkel 12 befestigt und ist mit einem oberhalb des Querbalkens 25 vorstehenden Daumengriff 21 a an ihrem oberen Ende fest verbunden. Der Zeiger 16a besitzt an seinem oberen Ende eine Pfeilmarkierung 23 welche auf einer Skala 22 des bogenförmigen Skalenteils 18 den Abstand der beiden Enden 26, 27 anzeigt. Wenn man mit der Hand den Griffteil 20 packt und mit dem Daumengriff 21 a die Biegefeder 15 zur Hand hinzieht, entsteht eine Vorspannung an den Enden 26, 27 der Schenkel 11, 12 die taktil einer Rückmeldung über das Anliegen der Enden 26, 27 an den Pedikelschrauben 3, 3' entspricht, wobei der Abstand der Enden unter einer vorgegebenen Vorspannung abgelesen werden kann, weil am Daumengriff 21 a eine Skala 24 angebracht ist an der die Vorspannung relativ zur Pfeilmarkierung 23 des Zeigers 16a ablesbar ist. Ein Vorteil der Einrichtung besteht darin, dass sie mit einer Hand betrieben werden kann und dem Operateur die zweite Hand für zusätzliche Manipulationen, wie beispielsweise dem Zurückhalten von im Weg stehenden Gewebeteilen, frei lässt.

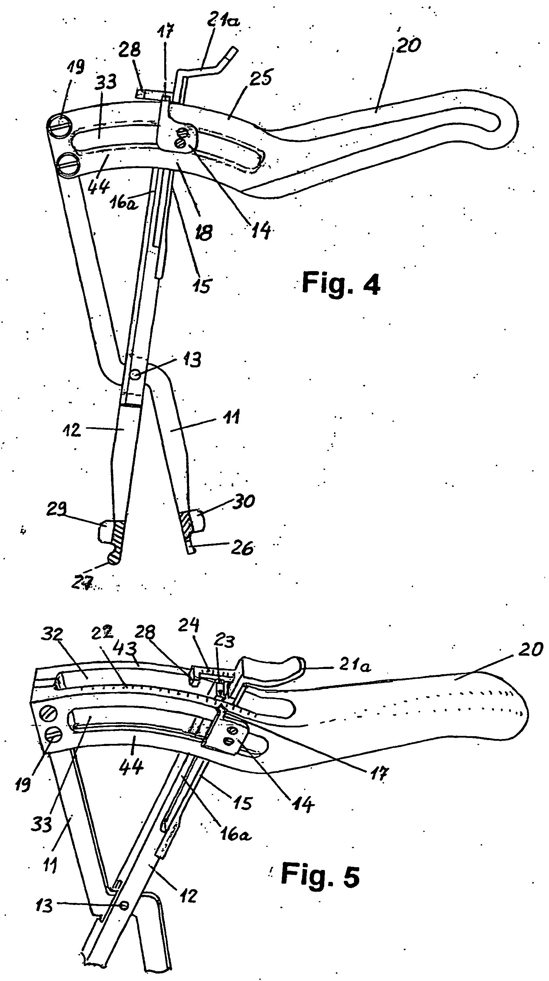

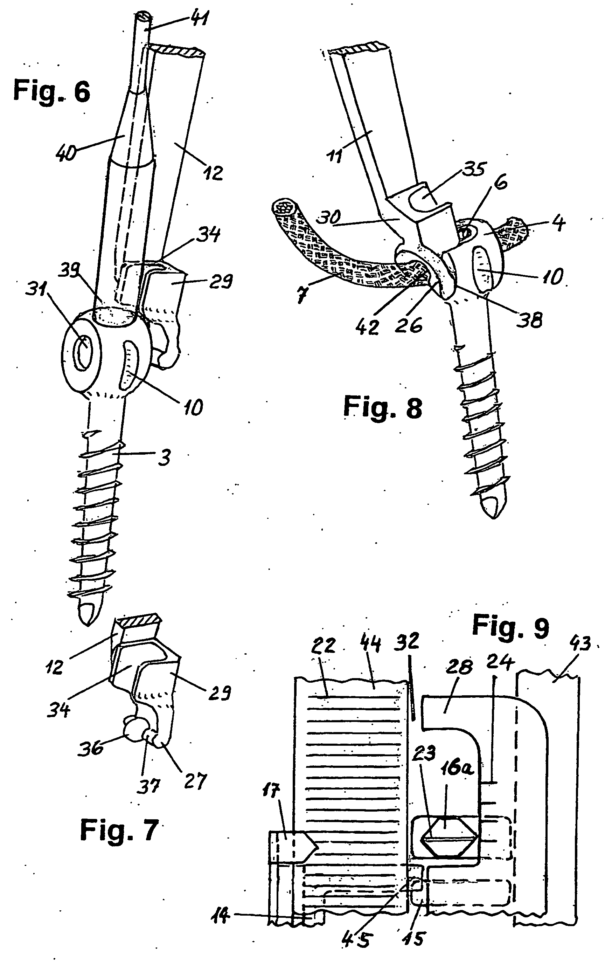

In den Figuren 4 bis 9 ist ein weiteres Ausführungsbeispiel gezeigt, in welchem weitere Verbesserungen zum Ausführungsbeispiel der Figur 3 enthalten sind. Es sind dabei gleiche Hinweiszeichen wie in Figur 3 verwendet worden. Der Längsschlitz 32 (Fig. 5 und 9) unterteilt den gekrümmten Skalenteil 18 des Querbalkens 25 in einen vorderen Teil 44 und in einen hinteren Teil 43. Im vorderen Teil 44 ist eine durchgehende, gekrümmte Nute 33 angebracht, in der ein Nutenstein 14, der selbst eine Reibungsbremse aufweist, verschieblich gelagert ist, um als Schleppzeiger 17 vom Zeiger 16a in Spreizrichtung mitgenommen zu werden. Dazu ist (Fig. 9) am Nutenstein 14 ein Nocken 45 angebracht, der in den Längsschlitz 32 hineinragt und vom Zeiger 16a mitgenommen wird. Der eigentliche Nutenstein 14 wird beispielsweise vom Längsschlitz 32 her eingesetzt und von vorne durch einen als Sicherungsplatte aufgeschraubten Schleppzeiger 17 gesichert. Beim Ziehen des Daumengriffs 21 a wird der Schleppzeiger 17 vom Zeiger 16a mitgenommen. Wenn nun die Enden 26, 27 auf den Widerstand der Pedikelschrauben stossen, kann die Biegefeder über den Daumengriff soweit vorgespannt werden bis eine vorgegebene Spannung erreicht ist. Bei dieser Spannung tritt auch der grösste Abstand zwischen den beiden Pedikelschrauben 3, 3' auf. Beim anschliessenden Entlasten des Daumengriffs bleibt der Schleppzeiger am Ort des grössten Abstandes stehen und speichert so den gemessenen Wert, ohne dass eine Beeinflussung beim weiteren Zusammenfahren der Spitzen 26, 27, beispielsweise für das Herauslösen von zentrierenden Vorsprüngen 36 aus den Durchgangsbohrungen 31 von Pedikelschrauben stattfindet.

Die Skala 24 für das Ablesen der Vorspannung ist an einem aus dem Daumengriff 21 a herausgebogenen Bügel 28 angebracht, welcher teilweise um den Zeiger 16a herumgeführt ist, um Überbelastungen der Biegefeder 15 zu verhindern dadurch, dass der Bügel 28 am Zeiger 16a anschlägt. Das heisst, der Zeiger 16a muss so stabil sein, dass er auch bei nicht fachmännischer Handhabung des Daumengriffs 21 a nicht plastisch deformiert wird. In den Figuren 6, 7 und 8 sind Alternativen im Bereich der Enden 26, 27 der Schenkel 11, 12 gezeigt. Beide Schenkel 11 und 12 sind zum Ende hin abgekröpft und haben zur Spreizrichtung seitlich versetzte Vorsprünge 29, 30 die jeweils das Ende bilden. Der seitliche Versatz hat den Vorteil, dass das ganze Abstandsmessgerät seitlich versetzt zu den Pedikelschrauben angeordnet ist und den Raum direkt über den Pedikelschrauben nicht für sich beansprucht. Im weiteren weisen die Vorsprünge 29, 30 in Spreizrichtung geöffnete Führungsgabeln 34, 35 auf, mit denen sie unter leichter Vorspannung entlang an Zentrierteilen 39 oder an röhrenförmigen Werkzeugen, welche am Kopf der Pedikelschrauben aufsitzen, von aussen bis an den Kopf der Pedikelschraube herangeführt werden können. Ein solches Zentrierteil 39 ist mit seinem unteren Ende in Figur 6 gezeigt. Es ist in dem für eine Klemmschraube 6 vorgesehene Gewinde eingeschraubt und verjüngt sich von einem zylindrischen Ansatz über einen Konus 40 bis zu einem biegeelastischen mittleren Teil 41. In Figur 8 ist das Band 7 bereits mit einer Klemmschraube 6 fixiert. Für die Fixierung kann ein röhrenförmiger Gegenhalter mit einem innen geführten Drehschrauber verwendet werden, der in einer Parallelanmeldung beschrieben ist. Ein solcher Gegenhalter stützt sich formschlüssig in Kerben 10 des Kopfes 4 ab und ist rohrförmig nach oben fortgesetzt. Wenn man diesen Gegenhalter nach dem Eindrehen der Klemmschraube 6 stehen lässt, kann man ihn wie das Zentrierteil 39 zur Führung von einem seitlichen Vorsprung 30 mit Führungsgabel 35 auf seinem Weg zur Pedikelschraube benutzen. Das Ende 26 ist zu einer Gabel 42 ausgebildet, die sich am eingezogenen Band 7 zentriert. Die Vorspannung zur Pedikelschraube wird über Druckzonen 38 aufgebracht welche beidseitig vom Band 7 an der Gabel 42 angeordnet sind.

In Figur 7 ist für den seitlichen Vorsprung 29 ein Halbkugelvorsprung 36 gezeigt, welcher sich in der Durchgangsbohrung 31 einer Pedikelschraube zentrieren kann. Beidseitig von dem Halbkugelvorsprung 36 sind Druckzonen 37 angeordnet, welche eine Vorspannkraft auf die Pedikelschraube 3 übertragen können.

### Teileliste

- 1: Rückenwirbel
- 2: Rückenwirbel
- 3: Pedikelschraube
- 3': Pedikelschraube
- 4: Kopf
- 5: Stirnfläche
- 6: Klemmschraube
- 7: Band
- 8 9: Bandscheibe
- 10: Kerbe
- 11: Schenkel
- 12: Schenkel
- 13: Drehachse
- 14: Nutenstein
- 15: Biegefeder
- 16: Zeiger
- 16a: Zeiger
- 17: Schleppzeiger
- 18: Skalenteil
- 19: Schraube
- 20: Griffteil
- 21: Vorsprung
- 21a: Daumengriff
- 22: Skala (Abstand)
- 23: Pfeilmarkierung
- 24: Skala (Vorspannung)
- 25: Querbalken
- 26: Ende
- 27: Ende
- 28: Bügel
- 29: seitlicher Vorsprung
- 30: seitlicher Vorsprung
- 31: Durchgangsbohrung
- 32: Längsschlitz
- 33: Nute
- 34: Führungsgabel
- 35: Führungsgabel
- 36: Halbkugelvorsprung
- 37: Druckzone
- 38: Druckzone
- 39: Zentrierteil
- 40: Konus
- 41: biegeelastischer Teil
- 42: Gabel
- 43: hinterer Teil
- 44: vorderer Teil
- 45: Nocken

## Patentansprüche

1. Abstandsmessgerät für Pedikelschrauben mit zwei sich an einer Drehachse (13) kreuzenden Schenkeln, die mit ihren Enden (26,27) an,zwei in benachbarten Rückenwirbeln (1, 2) eingedrehte Pedikelschrauben (3) ansetzbar sind, wobei ein erster Schenkel (11) an der entgegengesetzten Seite zu seinem Ende (26) in einen Querbalken (25) übergeht, welcher mit einem bogenförmigen Skalenteil (18) versehen ist, wobei der zweite Schenkel (12) einen über den Querbalken (25) vorstehenden Vorsprung (21) zur Verstellung aufweist und als Zeiger ausgebildet ist, um den Abstand zwischen zwei Pedikelschrauben mit dem Zeiger (16) an einer Skala (22) des Skalenteils anzuzeigen, **dadurch gekennzeichnet, dass** der zweite Schenkel (12) in einen Zeiger (16a) und eine parallel dazu verlaufende Biegefeder (15) unterteilt ist, an welcher ein ausgeprägter Daumengriff (21a) befestigt ist, und dass der Querbalken als Handgriff (25a) geformt ist, um über die Biegefeder (15) eine Spreizkraft an den Enden (26, 27) zu erzeugen, welche über eine mit dem Daumengriff (21a) verbundene Skala (24) relativ zum Zeiger (16a) ablesbar ist.

2. Abstandsmessgerät nach Anspruch 1, **dadurch gekennzeichnet, dass** der zweite Schenkel (12) mit Zeiger (16a) und Biegefeder (15) in einem Längsschlitz (32) des Querbalkens (25) beidseitig geführt ist.

3. Abstandsmessgerät nach einem der Ansprüche 1 oder 2, **dadurch gekennzeichnet, dass** der bogenförmige Skalenteil (18) eine Nute (33) mit einem Nutenstein (14) aufweist, welcher als Schleppzeiger (17) vom Zeiger (16a) mitnehmbar ist und ebenfalls den Abstand der Enden (26, 27) auf der Skala (22) aufzeigt, wobei er im maximal gemessenen Abstand verharrt.

4. Abstandsmessgerät nach einem der Ansprüche 1 bis 3, **dadurch gekennzeichnet, dass** eines der Enden (26, 27) als Gabel (42) ausgeführt ist, welche an einem durch den Kopf (4) einer Pedikelschraube (3) durchgezogenen Band (7) zentrierbar ist.

5. Abstandsmessgerät nach einem der Ansprüche 1 bis 3, **dadurch gekennzeichnet, dass** eines der Enden (26, 27) als Halbkugelvorsprung ausgeführt ist, welcher an einer Durchgangsbohrung (31) im Kopf (4) einer Pedikelschraube (3) zentrierbar ist.

6. Abstandsmessgerät nach einem der Ansprüche 1 bis 5, **dadurch gekennzeichnet, dass** die Enden (26, 27) jeweils an einem zum Schenkel (11, 12) seitlich versetzten Vorsprung (29, 30) angebracht sind.

7. Abstandsmessgerät nach Anspruch 6, **dadurch gekennzeichnet, dass** die Vorsprünge (29, 30) oberhalb der Enden (26, 27) jeweils eine in Spreizrichtung orientierte Führungsgabel (34, 35) aufweisen, mit der die Enden (26, 27) entlang einem im Kopf (4) einer Pedikelschraube (3) angeschraubtem Zentrierteil (39) an den Kopf (4) zuführbar sind.

8. Abstandsmessgerät nach einem der vorangehenden Ansprüche, **dadurch gekennzeichnet, dass** die Skala (24) für die Ablesung der Vorspannung an einem Bügel (28) angebracht ist, welcher den Zeiger (16a) hintergreift, damit eine Überbeanspruchung der Biegefeder (15) mit dem Daumengriff (21a) verhinderbar ist.

9. Abstandsmessgerät nach einem der vorangehenden Ansprüche, **dadurch gekennzeichnet, dass** die Schenkel (11, 12) aus Metall und der Querbalken (25) aus Kunststoff ausgeführt sind.

10. Abstandsmessgerät nach einem der Ansprüche 6 bis 9, **dadurch gekennzeichnet, dass** die seitlich versetzten Vorsprünge (29, 30) als Adapterstücke auf die Schenkel (11, 12) aufsteckbar sind.

## Claims

1. A distance measuring instrument for pedicle screws having two limbs which cross at an axis of rotation (13) and whose ends (25, 26) can be positioned at two pedicle screws (3) screwed into adjacent spinal vertebrae (1, 2), with a first limb (11) merging at the opposite side to its end (26) into a crossbar (25) which is provided with an arc-shaped scale part (18), with the second limb (12) having a projection (21) projecting over the crossbar (25) for the adjustment and being formed as a pointer to display the spacing between two pedicle screws with the pointer (16) at a scale (22) of the scale part, **characterised in that** the second limb (12) is divided into a pointer (16a) and into a flexural spring (15) extending parallel to it to which a pronounced thumb grip (21a) is fastened; and **in that** the crossbar is shaped as a handle (25a) to produce a spreading force at the ends (26, 27) via the flexural spring (15) which can be read off relative to the pointer (16a) via a scale (24) connected to the thumb grip (21a).

2. A distance measuring instrument in accordance with claim 1, **characterised in that** the second limb (12) with pointer (16a) and flexural spring (15) is guided at both sides in an elongate slot (32) of the crossbar (25).

3. A distance measuring instrument in accordance with any one of claims 1 or 2, **characterised in that** the arc-shaped scale part (18) has a groove (33) with a key (14) which can be taken along as a trailing pointer (17) by the pointer (16a) and likewise shows the spacing of the ends (26, 27) on the scale (22), with it remaining in place at the maximum spacing measured.

4. A distance measuring instrument in accordance with any one of claims 1 to 3, **characterised in that** one of the ends (26, 27) is designed as a fork (42) which can be centred at a band or cable (7) drawn through the head (4) of a pedicle screw (3).

5. A distance measuring instrument in accordance with any one of claims 1 to 3, **characterised in that** one of the ends (26, 27) is designed as a hemispherical projection which can be centred at a passage bore (31) in the head (4) of a pedicle screw (3).

6. A distance measuring instrument in accordance with any one of claims 1 to 5, **characterised in that** the ends (26, 27) are each attached to a projection (29, 30) laterally offset relative to the limb (11, 12).

7. A distance measuring instrument in accordance with claim 6, **characterised in that** the projections (29, 30) each have a guide fork (34, 35) above the ends (26, 27) and oriented in the spreading direction, with which the ends (26, 27) can be guided to the head (4) along a centring part (39) screwed on into the head (4) of a pedicle screw (3).

8. A distance measuring instrument in accordance with any one of the preceding claims, **characterised in that** the scale (24) for the reading of the pre-tension is attached to a hook (28) which engages behind the pointer (16a) so that an overstraining of the flexural spring (15) can be prevented by the thumb grip (21a).

9. A distance measuring instrument in accordance with any one of the preceding claims, **characterised in that** the limbs (11, 12) are made of metal and the crossbar (25) is made of plastic.

10. A distance measuring instrument in accordance with any one of claims 6 to 9, **characterised in that** the laterally offset projections (29, 30) can be pushed onto the limbs (11, 12) as adapter pieces.

## Revendications

1. Instrument de mesure d'écartement pour vis pédiculaires, comportant deux branches se croisant au niveau d'un axe de rotation (13), qui peuvent être fixées par leurs extrémités (26, 27) sur deux vis pédiculaires (3) vissées dans des vertèbres dorsales (1, 2) adjacentes, une première branche (11) se transformant du côté opposé à son extrémité (26) en une barre transversale (25) qui est munie d'une partie graduée (18) de forme cintrée, la deuxième branche (12) comportant pour le déplacement une saillie (21) qui dépasse de la barre transversale (25), et étant agencée en tant qu'aiguille afin d'indiquer avec l'aiguille (16) l'écartement entre deux vis pédiculaires sur une échelle graduée (22) de la partie graduée, **caractérisé en ce que** la deuxième branche (12) est subdivisée en une aiguille (16a) et en un ressort de flexion (15) s'y étendant parallèlement, sur lequel est fixée une manette de pouce (21a) faisant saillie, et **en ce que** la barre transversale est agencée sous la forme d'une poignée (25a) afin d'engendrer par l'intermédiaire du ressort de flexion (15) une force d'écartement au niveau des deux extrémités (26, 27), qui peut être lue par rapport à l'aiguille (16a) par l'intermédiaire d'une échelle graduée (24) reliée à la manette de pouce (21a).

2. Instrument de mesure d'écartement selon la revendication 1, **caractérisé en ce que** la deuxième branche (12) avec l'aiguille (16a) et le ressort de flexion (15) est guidée de part et d'autre dans une fente longitudinale (32) de la barre transversale (25).

3. Instrument de mesure d'écartement selon l'une des revendications 1 et 2, **caractérisé en ce que** la partie graduée (18) de forme cintrée comporte une rainure (33) avec un coulisseau (14) qui, en tant qu'aiguille entraînée (17), peut être entraîné par l'aiguille (16a), et qui indique également l'écartement entre les extrémités (26, 27) sur l'échelle graduée (22), étant précisé qu'il s'immobilise sur l'écartement maximal mesuré.

4. Instrument de mesure d'écartement selon l'une des revendications 1 à 3, **caractérisé en ce que** l'une des extrémités (26, 27) est réalisée sous la forme d'une fourche (42), qui peut être centrée sur une bande (7) passée à travers la tête (4) d'une vis pédiculaire (3).

5. Instrument de mesure d'écartement selon l'une des revendications 1 à 3, **caractérisé en ce que** l'une des extrémités (26, 27) est réalisée sous la forme d'une saillie hémisphérique, qui peut être centrée au niveau d'un perçage de passage (31) pratiqué dans la tête (4) d'une vis pédiculaire (3).

6. Instrument de mesure d'écartement selon l'une des revendications 1 à 5, **caractérisé en ce que** les extrémités (26, 27) sont respectivement fixées sur une saillie (29, 30) décalée latéralement par rapport aux branches (11, 12).

7. Instrument de mesure d'écartement selon la revendication 6, **caractérisé en ce que**, au-dessus des extrémités (26, 27), les saillies (29, 30) comportent chacune une fourche de guidage (34, 35) orientée dans le sens de l'écartement, avec laquelle les extrémités (26, 27) peuvent être amenées à la tête (4) le long d'un élément de centrage (39) vissé dans la tête (4) d'une vis pédiculaire (3).

8. Instrument de mesure d'écartement selon l'une des revendications précédentes, **caractérisé en ce que**, pour la lecture de la précontrainte, l'échelle graduée (24) est fixée sur un étrier (28) qui passe derrière l'aiguille (16a) afin qu'une sollicitation excessive du ressort de flexion (15) portant la manette de pouce (21a) puisse être évitée.

9. Instrument de mesure d'écartement selon l'une des revendications précédentes, **caractérisé en ce que** les branches (11, 12) sont réalisées en métal et la barre transversale (25) en matière plastique.

10. Instrument de mesure d'écartement selon l'une des revendications 6 à 9, **caractérisé en ce que** les saillies (29, 30) décalées latéralement peuvent être emboîtées sur les branches (11, 12) en tant que pièces d'adaptation.
